# EUROPEAN PATENT SPECIFICATION

(11) **EP 0 597 836 B1**
(45) Date of publication and mention of the grant of the patent: **18.06.1997**
(21) Application number: 91908126.5
(22) Date of filing: 22.04.1991
(51) Int. Cl.: C12N 11/14

(54) **SOLID SUPPORT MEDIUM FOR MICROBE PREPARATIONS AND A METHOD FOR CULTIVATION OF MICROBES**
FESTPHASENTRÄGER ZUR MIKROBENKULTUR UND VERFAHREN ZUR ZÜCHTUNG VON MIKROBEN
MILIEU SOLIDE DE SUPPORT POUR PREPARATIONS MICROBIENNES ET PROCEDE DE CULTURE DE MICROBES

(43) Date of publication of application: 25.05.1994
(73) Proprietor: KEMIRA OY, SF-02271 Espoo (FI)
(72) Inventor: SEISKARI, Pekka, SF-02400 Kirkkonummi (FI); PULKKANEN, Heikki, SF-01620 Vantaa (FI); VAPAAOKSA, Pekka, SF-28600 Pori (FI)
(74) Representative: Dost, Wolfgang, Dr.rer.nat., Dipl.-Chem.
(86) International application number: FI9100120
(87) International publication number: WO9218623

(56) References cited:
- EP-A- 0 267 470
- WO-A-85/00380
- DD-A- 141 978
- GB-A- 2 125 407
- US-A- 4 327 181
- US-A- 4 748 121
- Dialog Information Services, WPI, Accession No. 086-116381, FUJI DEVISUN KAGAKU: "Prepn. of cell cuitivation carrier involves adsorbing protein on porous silics gel material with gelatin coating"; & JP,A,61 019 568 860829 8641 (Basic).
- Dialog Information Services, WPI, Accession No. 082-E35982, HITACHI, SHIPBLD ENGG KK: "Alcohol prodn. by termentation using microbes adhered to porus crushed vermiculite carrier"; & JP,A,57 050 888 820325 8218 (Basic).
- CHEMICAL ABSTRACTS, Volume 77, No. 10, 4 September 1972, (Columbus, Ohio, US), LE DRED, R et al.: "Preparation of porous products by acid treatment of a mixed-layer mica-vermiculite mineral", see page 320, Abstract 66562f, & Bull. Groupe Fr. Argiles 1972, 24(1), 27-40.

## Description

The invention relates to a method for the culture of micro-organisms in a solid nutrient medium, wherein a silicate-based material is treated with a nutrient source, and one or more microbial species are added, and the produced mixture is kept under conditions in which the microbes will propagate. The invention also relates to a product which is made up of a mixture of a silicate-based material and one or more species of microbes.

The culture of microbes in solid culture media is an old method which is conventionally used especially in the food industry, for example in the production of blue cheeses and oriental koji-type preparations. These processes have been learned primarily through tradition, at first without any knowledge of their microbiological and enzymatic character.

In recent years there has been increasing awareness in the biotechnological industry that solid-state fermentation (SSF) processes constitute a noteworthy technique alongside submerged fermentation, which is the most commonly used. In many cases, SSF is the only possible process for the biotechnological production of a certain product, for example owing to the physiology of the growth of the microbe used. Often SSF is also a more economical process than submerged fermentation: the requirements of asepsis are not as high in certain applications, the process is simpler, and the costs of after-treatment are lower owing to the lower water content in the medium.

The usability of SSF is crucially dependent on the properties of the culture medium: nutrients, water-binding capacity, and structure. In conventional SSF, the media used are relatively complex materials which serve simultaneously as sources of nutrients, binders of water, and support materials. Examples of such materials include cereal grains, chaff, chopped straw, saw dust, peat, and compost (e.g. US Patent 4,748,021, FI Patent 69390). Sometimes components, such as gypsum or lime, which improve the structure of the medium are added to these media.

The industrial use of the culture media listed above is based partly on their economical price and good availability. In the developing of new biotechnological products, such as microbial inoculums, it is of primary importance that the properties of the final product be precisely those desired. For example, when cellulose-containing biomass is used as a medium in SSF, it is in practice almost impossible to obtain from a microbial inoculum a dry powder usable as a seed-dressing agent or spray powder.

It is also previously known to use various silicate minerals, such as vermiculite, bentonite and kaolinite as SSF support materials, especially in the preparation of inoculums of nitrogen bacteria and mycelia (e.g. Appl. Env. Microbiol. 53 (1987) 2138-2140; Curr. Sci. 51 (1982) 430-432). The possibilities of formulating such products for end use are, however, limited, and they are indeed in general used either as such, dried or most often undried, or in some way granulated.

In formulating preparations which contain live micro-organisms, efforts are made to bring them into a form as easily applicable as possible to each given purpose, usually either liquid concentrates or powders. For example in agricultural use it is advantageous if a microbial preparation can be sprayed at the target or be used for dressing plant seeds. This sets high requirements on the formulation of the microbial preparation, especially as regards the viability of the micro-organisms and the spraying and dressing properties of the preparation. Usually support materials, various protective substances, moisturizers, dispersing agents, and pH control agents are used in the formulation.

It is the purpose of the support material not only to protect the microbe but also to serve as a diluent; these supports are typically various organic natural materials, their derivatives, or inorganic minerals. The protective substances may be, for example, UV-protectants or anti-oxidants. The properties required of a support material include a suitable specific surface, a good buffering capacity, and a correct pH range.

It is previously known to use as a support material in the formulation of microbial preparations various inorganic silicate minerals such as vermiculite, kaolinite or montmorillonite (e.g. Can. J. Microb., Vol. 12 (1966), pp. 1235-1246). The quality of materials of this type may vary greatly, and furthermore, the microbe-inhibiting components possibly present in them may cause problems in the formulation of micro-organisms.

From the viewpoint of industrial production of microbial preparations it is necessary to be able to use in the culture or formulation an inexpensive, even-quality support material by means of which the desired advantageous properties can be obtained for the culture medium and the end product.

Some of the most important requirements set on industrial-scale SSF culture media are listed below:

Structure of the culture medium: The medium must be porous, preferably granular, so that the microbes will have a maximal growth surface per unit volume. A granular structure also ensures a sufficient supply of oxygen for the microbes.

Moisture content: The moisture content must be high enough to enable the microbes to grow in the medium. However, the quantity of free water in the medium should be minimal in order that water should not block the spaces in which air flows. A large water content will also increase the costs of drying and separation in the after-treatment.

Utilization of nutrients: For the economy of the process it is preferable that nutrients are present in precisely the quantity required by the microbe in order to grow and/or to produce the desired component at the desired rate.

After-treatment: The culture medium must be easy to introduce into the fermentor and to remove from it. In the case of microbial inoculums it is also necessary that the medium can be easily dried so that the microbes or their spores will survive the drying. After the drying it is often also necessary to be able to grind the product to a powder gently so that the organism will not suffer excessively.

Formulation: It is preferable that the medium contains components which promote the usability of the product (adhesion to seeds, non-caking, suspendable).

Price: The price of the culture medium must be reasonable in proportion to the price of the product.

In the present invention these objectives have been achieved with a new process and the use of a product which are characterized in what is stated in the characterizing clauses of the independent patent claims. It has thus been observed that silicon dioxide particles obtained by leaching cations from silicate minerals are excellently applicable as a support material for SSF and for microbial preparations which contain live microbes. By its surface properties silicon dioxide particles are particles which are hydrophilic and have a high specific surface. Its pore structure consists primarily of micro- and mesopore structures with a natural ability to retain water adsorbed onto its surfaces. The moisture is primarily in the pores, and so the spaces between the particles will remain dry.

Silicon dioxide particles of the correct type are usually obtained from a silicate mineral by leaching it with an acid. In the silicon dioxide particles the space left by cations is filled with hydrogen atoms and the surface becomes hydrophilic.

The leaching technique is affected by any lattice defects in the mineral; if use is made of these defects, the acid can be caused to penetrate into the silicate and to remove the cations. In Example 1 such a lattice defect is due to the fact that part of the iron is bivalent and part of it is trivalent. When the bivalent iron present in the lattice is oxidized to trivalent iron during the leach, the ion radius of the iron decreases and there is produced in the lattice more space, in which the acid has access to the cation structure to leach the cations without damaging the silicon frame of the silicate. In almost all natural silicates there are lattice defects with the help of which the silicate can be leached to produce silicon dioxide particles.

In the production of the product used according to the invention, the starting material consists of silicate particles, such as particles of phlogopite, biotite, vermiculite, bentonite or kaolinite. The silicate particles are preferably sedimentary silicate, particularly phlogopite or biotite. Synthetic mica can also be used.

When silicate particles are treated with an acid to remove the cations present in the silicate, the treatment acid is an aqueous acid solution having a concentration ranging from 0.7 to 70 % by weight, preferably from 4 to 65 % by weight. The corresponding pH range is in this case 0-7. The temperature range of the treatment is preferably approximately 0-100 °C at normal pressure. Some suitable acids are H₂SO₄, HCl, HNO₃, HF, HBr, HI, HClO₃, HBrO₃, HClO₄, H₃PO₄, citric acid, salicylic acid, tartaric acid, organic acids, oxalic acid, and mixtures of the above acids. The most preferred acids are sulfuric acid, hydrochloric acid and nitric acid.

The acid treatment can, when necessary, be carried out in the presence of an oxidizing or reducing additive. Examples of typical oxidizing additives include HNO₃, H₂O₂, MnO₄(-), CrO₇(2-), ClO₄(-), ClO₃, O₂, persulfates, Cl₂, Br₂. Examples of usable reducing agents include Sn(II), Fe(II), S₂O₃(-), Cu(I), aldehydes, ketones, H₂PO₄(-), sulfites, phosphites, V(II) and V(III). The amount of the oxidizing or reducing additive is dependent on the extent of effect it is desired to have on the solubility of the cations of the silicate. Typical additive amounts are 0-20 % of the weight of the initial material.

The density of the silicon dioxide particle material of the product used according to the present invention drops by approximately 30-50 % as a result of the acid leach. It can thus be seen that the product used according to the invention will be considerably lighter in weight than a product according to the state of the art, based on unleached silicate particles.

The preferred particle size range of the silicon dioxide particle material used in the product varies according to its use. A preferred particle size range is 1 - 2000 µm, the most preferred is 10 - 900 µm. The specific surface area is within a range of approximately 10-1000 m²/g, which makes the material usable for applications requiring a large specific surface.

The product used according to the invention comprises thus silicon dioxide particles with a microbial inoculum on its surface. The microbe may be, for example, a fungus, a bacterium, plant cells, or animal cells. The microbes may either be grown on the surface of the silicon dioxide particles by SSF or be mixed with the silica after culture, in which case the microbes will be grown separately, for example by submerged fermentation.

Any nutrient source, soluble or insoluble, for example chitin, is usable as a nutrient source in SSF. After culture in a static column reactor or mixed solid-state fermentation, and a possible drying, the product is ready for use. Pre- and after-treatment of the product (grinding, sterilization) is simple, since the basic properties of the silicon dioxide particles are retained through the treatment.

Owing to the granularity of the medium, the surface area for the microbial culture is large, and the transport of oxygen to the microbes is hindrance-free even in large reactors. The removing of the medium from the fermentor at the end of the culture is easy also in cases which require asepsis.

The drying of the medium, when necessary, with its microbes and/or microbe spores after the culture will also not cause difficulties. The porous structure of the silica will protect the microbes and, since the silica support has already been ground to fine powder in advance, gentle grinding of the product after the culture will not be detrimental to the microbes. The product thus obtained is excellent as a seed-dressing or spray powder.

Owing to its surface properties, the silicon dioxide particles serve effectively as a protective and support material for the microbes mixed with it. In addition, they prevent the product from caking.

The invention is described below in greater detail with the help of examples.

### Example 1

Preparation of silicon dioxide particles from phlogopite

| | | |
|---|---|---|
| a) | 700 g | phlogopite, particle size 0.01-1500 µm |
| | 2.5 l | water |
| | 2065.36 g | H₂SO₄ (96 %) |
| | 331.33 g | H₂O₂ |

A slurry is prepared from the mica, water and sulfuric acid, the slurry is heated to 95 °C, whereafter H₂O₂ is fed under the surface for approximately 5 hours under constant mixing. The total leaching time is 7 hours, of which approximately one hour, before the feeding in of the H₂O₂, consists only of mixing and heating, and one hour, after the end of the feeding in of the H₂O₂, consists of mixing. Thereafter the mother liquor is filtered off and the remainder (the silicon dioxide particles) is washed several times, slurrying it between washes, clean of acid and cation residues.

| | | |
|---|---|---|
| b) | 700 g | phlogopite, particle size 0.01-1500 µm |
| | 2.5 l | water |
| | 750 g | nitric acid (100 %) |

The leach is carried out as above, except that oxidant is not needed, since nitric acid in itself has an oxidizing effect.

### Example 2

The procedure is in other respects the same as in Example 1, but anortisite is used instead of phlogopite.

### Example 3

| | |
|---|---|
| 600 g | serpentinite |
| 3 l | ion-exchanged water |
| 1770.31 g | H₂SO₄ (96 %) |
| 161.82 g | HNO₃ (65 %) |

The leaching temperature was 95-97 °C and the leaching time was 7 hours. The temperature was raised to 95 °C, and HNO₃ was introduced slowly under the surface for 5 hours. After the ending of the feed the mixture was mixed for 2 hours before filtration. The mother liquor was filtered off, the cake was slurried in a 1 % H₂SO₄ solution, and was allowed to stand in it for 1-3 days. The cake was slurried and washed five times with ion-exchanged water. The pH of the final filtrate was 4.1.

### Example 4

The procedure was in other respects as in the previous example, but the mineral used was vermiculite or wollastonite.

### Example 5

### Culture of fungicidal fungi in a solid silica medium

1 g of finely-ground chitin was mixed with 9 g of a salt solution having the following composition:

| | |
|---|---|
| 1 g/l | KH₂PO₄ |
| 0.2 g/l | MgSO₄*7H₂O |

The mixture was sterilized (20 min, 120 °C), and the cooled mixture was inoculated with 1 ml of a suspension of spores of fungicidal Trichoderma sp.; the suspension had been obtained by scraping the spores from a PDA dish into sterile, distilled water.

The chitin slurry inoculated with Trichoderma was mixed with 6 g of a finely-ground silicon dioxide particles prepared according to Example 1 from phlogopite, the culture medium thus becoming granular. The medium was incubated at room temperature (20 °C) for 7 days, until the medium was covered throughout by fungus mycelium and spores.

The medium, spread into a thin layer on a Petri dish, was dried for one day at room temperature. The dry product was ground into powder in a mortar. The concentration of spores in the product was 2.4*10⁹/g.

The concentration of spores in a Gliocladium sp. preparation prepared in a respective manner was 4.8*10⁸/g.

### Example 6

Fungicidal Streptomyces griseoviridis actinomycete was grown in a solid culture medium the composition of which was:

| | |
|---|---|
| silica | 10 g |
| condensed starch mash* | 3.6 g |
| dolomite lime | 2 g |
| water | 13.4 g |

| | |
|---|---|
| * Oy Alko Ab, Rajamäki, solids content approx. 36 % | |

The medium was autoclaved and inoculated with 1 ml of an actinomycete spore suspension. The culture took place at 28 °C for 7 days. The medium which had grown full was dried at room temperature. The concentration of spores in the product was 3*10⁹/g.

### Example 7

Phlemia gigantea fungus intended for combatting Fomes annosum was cultured on the same medium as the actinomycete. After 11 days of culture the medium was dried at room temperature. The concentration of spores in the product was 2.5*10⁷/g.

### Example 8

### Culture of insecticidal fungi in a solid silica medium

1 g of finely-ground chitin was mixed with 9 g of a salt solution having the following composition:

| | |
|---|---|
| 1 g/l | K₂HPO₄*3H₂O |
| 0.2 g/l | MgSO₄*7H₂O |

The mixture was sterilized (20 min, 120 °C), and the cooled mixture was inoculated with 1 ml of a spore suspension of insecticidal Beauveria bassiana; the suspension had been obtained by scraping the spores from a PDA dish into sterile, distilled water.

The mixing with the silica support, the culture and the drying were carried out as with Trichoderma in Example 5. The concentration of spores in the dried and ground product was 3.4*10⁸.

The concentration of spores in an insecticidal Metarrhizium anisopliae preparation cultured in a respective manner was 1.5*10⁸/g.

### Example 9

### Formulation of a fungicidal actinomycete preparation on silica

Streptomyces griseoviridis actinomycete was cultured in a whey medium (10 g/l whey protein), and the growth was separated by centrifugation. 9.0 g of saccharose and 15.0 g of a finely-ground silica prepared according to Example 1 were mixed with 5.1 g of separated cell dry matter. The mixture was lyophilized and ground. The viability obtained for the dry product was 5.9*10⁷ CFU/g. After six weeks of storage at 28 °C the viability was 1.4*10⁷ CFU/g.

### Example 10

### Formulation of a fungicidal Bacillus preparation on silica

Bacillus pumilus was cultured in a medium having the composition:

| | |
|---|---|
| glucose | 4 g/l |
| yeast extract | 4 g/l |
| malt extract | 1 g/l |

The growth was separated by centrifugation, and 2.2 g of saccharose and 3.6 g of a finely-ground silica prepared according to Example 1 were mixed with 1.3 g of the separated cell dry matter. After lyophilization and grinding the viability was 6*10⁹ CFU/g and after ten weeks of storage at 28 °C it was 2.6*10⁸.

### Example 11

### Formulation of a fungicidal yeast preparation on silica

Cryptococcus macerans yeast was cultured in a potato dextrose medium. The growth was separated by centrifugation, and 4.0 g of saccharose and 11.7 g of a finely-ground silica prepared according to Example 1 were mixed with 11.8 g of the separated cell pulp. The mixture was lyophilized and ground. The viability obtained for the product was 9*10⁴ CFU/g.

## Claims

1. A solid state fermentation method for the culture of microbes in a solid culture medium, wherein a silicate-based material is treated with a nutrient source, and one or more microbe species are added, and the produced mixture is kept under conditions in which the microbes will propagate, **characterized** in that hydrophilic, substantially cation-free and porous silica is used as the silicate-based material.

2. A method according to Claim 1, **characterized** in that the hydrophilic, substantially cation-free and porous silica has been prepared by treating phlogopite, biotite, vermiculite, bentonite or kaolinite with an acid, possibly in the presence of an oxidizing or reducing additive, in order to substantially eliminate the cations present in the said silicates.

3. A method according to Claim 1 or 2, **characterized** in that the hydrophilic, substantially cation-free and porous silica is in a gel form.

4. A method according to Claim 1, 2 or 3, **characterized** in that the nutrient source is adsorbed onto the hydrophilic, substantially cation-free, porous silica.

5. A method according to any of the above claims, **characterized** in that the microbe species are fungi or bacteria.

6. Use of a product comprising a silicate-based material consisting of hydrophilic, substantially cation-free and porous silica, and one or more microbe species, in the method of any of claims 1 to 5.

7. The use according to claim 6, characterized in that the hydrophilic, substantially cation-free and porous silica is obtained from phlogopite, biotite, vermiculite, bentonite or kaolinite by treating with acid.

8. The use according to claim 6 or 7, characterized in that the hydrophilic, substantially cation-free and porous silica is based on silica particles having a particle size less than 2000 µm, preferably less than 900 µm.

9. The use according to any of claims 6 to 8, characterized in that the microbe species are fungi or bacteria.

## Patentansprüche

1. Festzustandsfermentationsverfahren zum Züchten von Mikroben in einem festen Kulturmedium, wobei ein auf Silicat basierendes Material mit einer Nährstoffquelle behandelt wird, eine oder mehrere Mikrobenspezies zugegeben werden und das gebildete Gemisch unter Bedingungen gehalten wird, unter denen die Mikroben sich vermehren, dadurch gekennzeichnet, daß hydrophiles, im wesentlichen von Kationen freies und poröses Siliciumdioxid als das Material auf Silicatbasis verwendet wird.

2. Verfahren nach Anspruch 1, dadurch gekennzeichnet, daß das hydrophile, im wesentlichen von Kationen freie und poröse Siliciumdioxid durch Behandlung von Phlogopit, Biotit, Vermiculit, Bentonit oder Kaolinit mit einer Säure, möglicherweise in Gegenwart eines oxidierenden oder reduzierenden Additivs, behandelt worden ist, um im wesentlichen die in den Silicaten vorhandenen Kationen zu beseitigen.

3. Verfahren nach Anspruch 1 oder 2, dadurch gekennzeichnet, daß das hydrophile, im wesentlichen von Kationen freie und poröse Siliciumdioxid in einer Gelform vorliegt.

4. Verfahren nach Anspruch 1, 2 oder 3, dadurch gekennzeichnet, daß die Nährstoffquelle auf dem hydrophilen, im wesentlichen von Kationen freien, porösen Siliciumdioxid adsorbiert ist.

5. Verfahren nach einem der vorstehenden Ansprüche, dadurch gekennzeichnet, daß es sich bei den Mikrobenspezies um Pilze oder Bakterien handelt.

6. Verwendung eines Produkts, das ein Material auf Silicatbasis, das aus hydrophilem, im wesentlichen von Kationen freiem und porösem Siliciumdioxid besteht, und eine oder mehrere Mikrobenspezies umfaßt, bei dem Verfahren nach einem der Ansprüche 1 bis 5.

7. Verwendung nach Anspruch 6, dadurch gekennzeichnet, daß das hydrophile, im wesentlichen von Kationen freie und poröse Siliciumdioxid aus Phlogopit, Biotit, Vermiculit, Bentonit oder Kaolinit durch Behandlung mit Säure erhalten wird.

8. Verwendung nach Anspruch 6 oder 7, dadurch gekennzeichnet, daß das hydrophile, im wesentlichen von Kationen freie und poröse Siliciumdioxid auf Siliciumdioxidteilchen mit einer Teilchengröße von weniger als 2000 µm und vorzugsweise von weniger als 900 µm basiert.

9. Verwendung nach einem der Ansprüche 6 bis 8, dadurch gekennzeichnet, daß es sich bei den Mikrobenspezies um Pilze oder Bakterien handelt.

## Revendications

1. Procédé de fermentation à l'état solide pour la culture de microbes dans lequel on traite un matériau à base de silicate par une source nutritive, et dans lequel on ajoute une ou plusieurs espèces de microbes, on garde le mélange obtenu dans des conditions dans lesquelles les microbes vont se développer, caractérisé en ce qu'on utilise de la silice hydrophile, en grande partie dépourvue de cations et poreuse, comme matériau à base de silicate.

2. Procédé selon la revendication 1, caractérisé en ce que l'on a préparé la silice hydrophile, essentiellement dépourvue de cations et poreuse, en traitant de la phlogopite, de la biotite, de la vermiculite, de la bentonite ou de la kaolinite avec un acide, avec la présence possible d'un additif oxydant ou réducteur, de façon à éliminer essentiellement les cations présents de ces silicates.

3. Procédé selon la revendication 1 ou 2, caractérisé en ce que la silice hydrophile, en grande partie dépourvue de cations et poreuse, est sous la forme d'un gel.

4. Procédé selon la revendication 1, 2, ou 3 caractérisé en ce que la source nutritive est adsorbée sur la silice hydrophile, dépourvue de cations, poreuse.

5. Procédé selon l'une quelconque des revendications ci-dessus, caractérisé en ce que les espèces de microbes sont des champignons ou des bactéries.

6. Utilisation d'un produit comprenant un matériau à base de silicate qui consiste en de la silice hydrophile, en grande partie dépourvue de cations et poreuse et, une ou plusieurs espèces de microbes, dans le procédé selon l'une quelconque des revendications 1 à 5.

7. Utilisation, selon la revendication 6, caractérisée en ce que la silice hydrophile, en grande partie dépourvue de cations et poreuse est obtenue à partir de la phlogopite, de la biotite, la vermiculite, de la bentonite ou de la kaolinite en la traitant avec un acide.

8. Utilisation, selon la revendication 6 ou 7, caractérisés en ce que la silice hydrophile, essentiellement dépourvue de cations et poreuse, est à base de particules de silice ayant une taille de particules de moins de 2 000 µm, de préférence de moins de 900 µm.

9. Utilisation, selon l'une quelconque des revendications de 6 à 8 caractérisée en ce que les espèces de microbes sont des champignons ou des bactéries.
